# EUROPÄISCHE PATENTSCHRIFT

(11) **EP 1 119 337 B1**
(45) Veröffentlichungstag und Bekanntmachung des Hinweises auf die Patenterteilung: **02.11.2006**
(21) Anmeldenummer: 99952461.4
(22) Anmeldetag: 22.09.1999
(51) Int. Cl.: A61K 6/02, A61K 6/083

(54) **DENTALWERKSTOFF AUFWEISEND PORÖSE GLASKERAMIKEN**
DENTAL MATERIAL WITH POROUS GLASS CERAMICS
MATERIAU DENTAIRE CONTENANT DES VITROCERAMIQUES POREUSES

(30) Priorität: 09.10.1998 DE 19846556
(43) Veröffentlichungstag der Anmeldung: 01.08.2001
(73) Patentinhaber: DeguDent GmbH, 63457 Hanau-Wolfgang (DE); Forschungszentrum Karlsruhe GmbH, 76133 Karlsruhe (DE)
(72) Erfinder: ALKEMPER, Jochen, D-68519 Viernheim (DE); RENTSCH, Harald, D-63457 Hanau (DE); DERMANN, Klaus, D-61231 Bad Nauheim (DE); RITZHAUPT-KLEISSL, Hans-Joachim, D-69190 Walldorf (DE); HAUSSELT, Jürgen, D-76726 Germersheim (DE); ALBERT, Philipp, D-63456 Hanau (DE); GALL, Corinna, D-36381 Schlüchtern (DE)
(74) Vertreter: Stoffregen, Hans-Herbert
(86) Internationale Anmeldenummer: PCT/EP1999/007057
(87) Internationale Veröffentlichungsnummer: WO 2000/021488

(56) Entgegenhaltungen:
- EP-A- 0 048 681
- EP-A- 0 434 334
- EP-A- 0 832 636
- EP-A- 0 839 511

## Beschreibung

Die vorliegende Erfindung richtet sich auf einen Dentalwerkstoff sowie ein Verfahren zu dessen Herstellung. Ebenso betrifft die Erfindung poröse Glaskeramiken, ein Verfahren zu deren Herstellung und Verwendung.

Insbesondere ist die Erfindung auf Dentalwerkstoffe auf Basis von polymerisierbaren, beispielsweise ethylenisch ungesättigten Monomeren, Epoxiden, Ormoceren, flüssigkristallinen Monomeren, Oxethanen, Spiroorthoester bzw. -carbonaten als Bindemittel, eines Katalysators für die Kalt-, Heiß- und/oder Photopolymerisation und bezogen auf den Dentalwerkstoff 20-70 Gew.-% eines anorganischen Füllstoffs (A) sowie bezogen auf den Dentalwerkstoff 0-60 Gew.-% von weiteren Füllstoffen (B) sowie 0-2 Gew.-% von weiteren üblichen Zuschlagstoffen gerichtet.

Auf Grund der möglichen Gesundheitsgefahren durch quecksilberhaltige Materialien (Amalgame) im Bereich der Zahnrestauration ist man verstärkt auf der Suche nach neuen quecksilberfreien Zubereitungen für diesen Anwendungszweck.

Aus der US 5426082 sind poröse Glaskeramiken bekannt, welche für die Herstellung von speziellen Katalysatoren dienen. Die dort genannten Keramiken sollen eine Mindestporenvolumen von >2000 mm³/g aufweisen. Diese hohen Porenvolumina sind für den Einsatz dieser Stoffe als Füllmaterialien in Dentalwerkstoffen nicht geeignet, da sie eine geringe Festigkeit der Füllungen bedingen.

Bei den in der EP 48 681 beschriebenen befüllten anorganischen porösen Teilchen handelt es sich um aus amorphen Gläsern bestehenden Füllstoffen. Die hier genannten Dentalwerkstoffe haben jedoch den Nachteil, daß die Partikel des benutzten Füllstoffs aufgrund ihrer Struktur und Größe lungengängig sind und so bei Implementierung die Gefahr einer der Asbestose vergleichbaren Krankheit besteht.

Die EP-A 0 530 926 offenbart Dentalmassen aus einem polymerisierbaren Monomer und einem anorganischen Füller, der zu 20 bis 80 Gew.-% aus sphärischen anorganischen Oxidpartikeln mit einer durchschnittlichen Teilchengröße zwischen 1,0 und 5,0 µm und zu 80 - 20 Gew.-% aus sphärischen anorganischen Oxidpartikeln mit einer Teilchengröße im Bereich von mindestens 0,05 µm und weniger als 1,0 µm besteht, wobei wenigstens 5 Gew.-% der letztgenannten Komponente im Bereich von 0,05 bis 0,2 µm liegen. Bei den anorganischen Partikeln handelt es sich ausschließlich um kugelförmige Partikel anorganischer Oxide des Siliciums, Zirkoniums, Aluminiums und Titans oder Mischoxide von Metallen aus der I - IV Hauptgruppe des Periodensystems mit Silicium. Die kugelförmigen Partikel werden z. B. durch hydrolytische Polymerisation von Alkoxysilanen hergestellt und können z. B. auch mit γ-Methacryloxypropyltrimethoxysilan oberflächenbehandelt werden.

In der DE 196 15 763 werden mit Monomeren beladene amorphe Siliziumdioxid-Gläser in Dentalkompositen beschrieben.

Die nach diesen letztgenannten Dokumenten aus Füllstoffen herstellbaren Zahnrestaurationsmaterialien auf Kunststoffbasis sind den Amalgamen in mehrfacher Hinsicht unterlegen:
- die Füllmaterialien sind nicht abrasionsfest genug, um im Kaubereich der Zähne verwendet zu werden
- die normalerweise in die Polymermischungen einpolymerisierten nichtporösen Materialien, wie z. B. Glaspartikel, müssen mit speziellen Kopplungsreagenzien überzogen werden und sind oftmals anfällig für hydrolytische Spaltungen zwischen Polymermaterial und Füllstoff. Dies verringert den Abrasionswiderstand durch Herausbrechen der Füllstoffe.
- Füllmaterialien, welche auf porösen amorphen Gläsern beruhen haben den Nachteil, daß die Füllmaterialien nicht röntgenopak sind sowie einen Brechungsindex aufweisen, der von der umgebenden Polymermatrix um mehr als 0,02 verschieden ist, wodurch trübe, wenig transparente Dentalwerkstoffe resultieren, welche durch Licht in einem Schritt schlecht härtbar sind und so schichtweise aufwendig in die Kavität einpolymerisiert werden müssen.

Aufgabe der vorliegenden Erfindung war es deshalb, Dentalwerkstoffe bereitzustellen, die die Nachteile der Materialien des Standes der Technik nicht aufweisen, insbesondere bei vergleichbar gutem Polymerisationsschrumpf eine erhöhte Festigkeit und verbesserte Abrasionsfestigkeit aufweisen. Zudem sollte die durch hydrolytische Spaltung bedingte Ablösung der Polymermatrix vom anorganischen Füllstoff unterbunden werden. Der Dentalwerkstoff sollte darüber hinaus falls gewünscht röntgenopak sein sowie derart transparent, daß man in einem Arbeitsschritt den Dentalwerkstoff in die Kavität des Zahnes einbringen und mit Licht aushärten kann.

Eine weitere Aufgabe der Erfindung war die Bereitstellung von bestimmten Glaskeramiken, welche sich als poröse Füllstoffe für den Einsatz in erfindungsgemäßen Dentalmaterialien eignen.

Die Aufgabe, sofern sie sich auf einen Dentalwerkstoff bezieht, wird gelöst durch einen Dentalwerkstoff mit den kennzeichnenden Merkmalen des Anspruchs 1. Die auf Anspruch 1 rückbezogenen Unteransprüche 2-10 stellen besondere Ausführungsformen des Dentalwerkstoffs unter Schutz. Ansprüche 11-15 stellen ein Verfahren zur Herstellung des Dentalwerkstoffs vor.

Die Aufgabe zur Angabe einer porösen Glaskeramik wird durch Anspruch 16 gelöst. Ansprüche 17-22 geben besondere Ausgestaltungen der Glaskeramiken an. Ansprüche 23-27 stellen ein Verfahren zur Herstellung der porösen Glaskeramik unter Schutz, Ansprüche 28-29 beschreiben die Verwendungen erfindungsgemäßer Glaskeramiken.

Dadurch, daß ein Dentalwerkstoff auf Basis von polymerisierbaren, beispielsweise ethylenisch ungesättigten Monomeren, Epoxiden, Ormoceren, flüssigkristallinen Monomeren, Oxethanen, Spiroorthoester bzw. -carbonaten als Bindemittel, eines Katalysators für die Kalt-, Heiß-und/oder Photopolymerisation und bezogen auf den Dentalwerkstoff 20-70 Gew.-% eines anorganischen Füllstoffs (A) sowie bezogen auf den Dentalwerkstoff 0-60 Gew.-% von weiteren Füllstoffen (B) sowie 0-2 Gew.-% von weiteren üblichen Zuschlagstoffen, wobei der anorganische Füllstoff (A) eine poröse Glaskeramik ist, deren Mikro- und/oder Mesoporen mit polymerisierbaren, ethylenisch ungesättigten Monomeren, Epoxiden, Ormoceren, flüssigkristallinen Monomeren, Oxethanen, Spiroorthoester bzw. -carbonaten oder mit deren polymerisierter Form beladen sind und der anorganische Füllstoff (A) zusätzlich ein kristallines Oxid mit einer Konzentration aufweist, dass der Brechungsindex des Füllstoffs (A) an den Brechungsindex der Polymerumgebung anpassbar ist und der anorganische Füllstoff (A) ein Porenvolumen größer als 200 und kleiner als 2000 mm³/g aufweist, bereitgestellt wird, ergibt sich ein ggf. röntgenopaker Dentalwerkstoff, dessen Transparenz gezielt auf die jeweiligen Bedingungen einstellbar ist und der bei mit den Materialien des Standes der Technik vergleichbar gutem Polymerisationsschrumpf eine verbesserte Festigkeit bei gleichzeitig verbesserter Abrasionsfestigkeit aufweist.

Die Porengröße (Porendurchmesser/-weite) des Füllstoffs (A) ist mithin verantwortlich dafür, wie gut die Monomeren in den Füllstoff (A) gelangen können. Bevorzugt ist ein Dentalwerkstoff, dessen glaskeramischer Füllstoff (A) eine Porengröße von größer als 1 nm und kleiner als 1000 nm aufweist. Besonders bevorzugt liegt die Porengröße des Füllstoffs (A) zwischen 10 nm und 100 nm.

Dadurch läßt sich eine vollständige Befüllung der Poren mit den polymerisierbaren Monomeren und gleichzeitig durch die im Vergleich mit der Wellenlänge des sichtbaren Lichts kleinen Dimensionen der Poren eine hohe Transparenz erzielen (keine Interferenzeffekte).

Die Partikelgröße des Füllstoffs (A) kann erfindungsgemäß zwischen 1 und 50 µm liegen, besonders bevorzugt zwischen 10 und 30 µm.

Das Porenvolumen des Füllstoffs (A) ist eine essentielle Charakteristik zur Herstellung des Dentalwerkstoffes. Das Porenvolumen darf nicht zu groß sein, da sonst der innere Zusammenhalt der Glaskeramik abnimmt. Ist es zu klein, nimmt die Haftung zwischen Füllstoff und Matrix ab, so daß die Oberfläche des Dentalwerkstoffs sehr rauh ausfällt und sich der Verschleiß erhöht. Aus diesen Gründen sollte das Porenvolumen des Füllstoffs (A) größer als 200 mm³/g und kleiner als 2000 mm³/g sein. Bevorzugt ist eine Größe zwischen 500 mm³/g und 1500 mm³/g.

Der glaskeramische Füllstoff (A) ist aus einem amorphen Glasanteil aufgebaut, in den kristalline Bereiche eingebettet sind, welche aus Oxiden der Metalle der 1. bis 4. Hauptgruppe sowie aus Oxiden der Metalle der Nebengruppen bestehen können.

Der Anteil der Oxide, die einzeln oder im Gemisch im Füllstoff (A) vorkommen können, kann zwischen 1 und 50 Gew.-% bezogen auf den Füllstoff (A) liegen.

Im auspolymerisierten Zustand kann das Verhältnis der Gew.-Teile von Polymer zu Füllstoff (A) zu Füllstoffen (B) der erfindungsgemäßen Dentalfüllungen im Verhältnisbereich von 10-80 zu 20-70 zu 1-30, bevorzugt 30-50 zu 30-60 zu 5-20 liegen (in Summe 100).

Gleichsam können bacterizide Mittel in den Füllstoff (A) implementiert werden. Als bacterizide Mittel sieht man insbesondere Hesperedin, Naringenin, Quercetin, Anisic Acid, Acetyl Coumarin, Sitosterol, Caryophyllen und Caryophyllenoxid an. Diese Verbindungen können nach den in US 4,925,660 beschriebenen Verfahren in die Poren des Füllstoffs (A) eingebaut werden.

Bevorzugt kann die innere und/oder äußere Oberfläche des mikro- und/oder mesoporösen glaskeramischen Füllstoffs (A) sowie die äußere Oberfläche des Füllstoffs (B) vor der Anwendung im Dentalwerkstoff optional mit dem Fachmann geläufigen, Oberflächenmaterialien chemisch modifiziert werden. Dies dient u. a. zur
a) Erhöhung der mechanischen Stabilität und Hydrophobizität
   und
b) weiteren Verbesserung der Kopplung des anorganischen Füllers an die organische Matrix.

In besonderer Ausführungsform ist der Füllstoff (A und/oder B) mit Silanen der allgemeinen Formel RSi(OX)₃, worin R eine Alkylgruppe mit 1 bis 18 C-Atomen und X eine Alkylgruppe mit 1 oder 2 C-Atomen ist, und/oder Metalloxiden nachbeschichtet. Zur Erhöhung der Stabilität und Hydrophobizität wird insbesondere Trimethylchlorosilan verwendet, wie beschrieben in Koyano, K. A.; Tatsumi, T.; Tanaka, Y.; Nakata, S. J. Phys. Chem. B 1997, 101, p. 9436 und Zhao, X. S.; Lu, G. Q., J. Phys. Chem. B 1998, 102, p. 1156. Wenn zur Nachbeschichtung ein Silan verwendet wird, ist es zweckmäßig, wenn dies in einer Menge von etwa 0,02 bis 2 Gewichtsprozent des Silans, berechnet als SiO₂, angewendet wird, bezogen auf das Gewicht des Füllstoffs (A) bzw. (B).

An der erfindungsgemäßen Dentalmasse ist vorteilhaft, daß die Farbe, Transparenz und Röntgenopazität alleine schon durch die Zusammensetzung des Füllstoffs (A) eingestellt werden kann. Optional können jedoch auch weitere Metalloxide zur Nachbeschichtung eingesetzt werden, wobei dies vorzugsweise in einer Menge von 1 bis 100 Gewichtsprozent, bevorzugt 10 Gewichtsprozent, bezogen auf den Metalloxidgehalt der nicht nachbeschichteten Füllstoffe (A) bzw. (B) geschieht.

Zu besonders vorteiligen Nachbeschichtungsagenzien gehören unter anderem (CH₃)₃SiCl, Methyltriethoxysilan, Ethyltriethoxysilan, Octyltriethoxysilan, Octadecyltriethoxysilan, Mono- oder Polyfluoralkylethoxysilan oder auch Silane mit funktionalisierten Organogruppen, die eine spätere weitere Modifizierung durch kovalente Bindungsknüpfung in bekannter Weise ermöglichen. Im letzteren Fall sind solche Organotrialkoxysilane im Hinblick auf den erfindungsgemäßen Einsatz der Partikel als Füllstoffe in polymeren oder polymerisierbaren Systemen bevorzugt, die solche funktionelle Gruppen aufweisen, mit denen sich eine kovalente Einbindung in das Polymermaterial erreichen läßt. Beispiele hierfür sind Trimethoxyvinylsilan, H₂C=C(CH₃)CO₂(CH₂)₃Si(OCH₃)₃, Triethoxyvinylsilan und 3-Glycidoxypropyltrimethoxysilan, sowie Silane mit Hydroxyl-, Carboxyl-, Epoxy- und Carbonsäureestergruppen tragenden anorganischen Resten. Die Einbindung der solcher Art modifizierten Partikel in den Dentalwerkstoff geschieht dabei durch Einarbeitung der Partikel in den Dentalwerkstoff und anschließende Polymerisation bei der eigentlichen, Aushärtung des Dentalwerkstoffes.

Bevorzugte Metalloxide, welche für die Nachbeschichtung eingesetzt werden, sind TiO₂, Fe₂O₃ und/oder ZrO₂.

Zweckmäßig ist auch eine Ausführungsform, in der der Füllstoff (A) bzw. (B) zusätzlich mit einer Schicht aus einem polymerisierbaren organischen Bindemittel überzogen ist, welches auf mono- oder mehrfachfunktionellen (Meth)acrylaten und/oder Reaktionsprodukten aus Isocyanaten und OH-gruppenhaltigen Methacrylaten beruht.

Dentalwerkstoff bezeichnet im Rahmen der Erfindung Materialien für die Zahnrestauration, wie Zahnfüllung, Inlays oder Onlays, Befestigungszemente, Glasionomerzemente, Kompomere, Verblendmaterialien für Kronen und Brücken, Materialien für künstliche Zähne, in Dentinbondings, Unterfüllmaterialien, Wurzelfüllmaterialien oder sonstige Materialien für die prothetische, konservierende und präventive Zahnheilkunde. Insbesondere fallen unter den Begriff Dentalwerkstoff auch Komposits für zahnmedizinische und zahntechnische Einsatzzwecke, Versieglermaterialien, selbsthärtende Komposits, Stumpfaufbaumaterialien, Verblendkunststoffe, hoch- und normalgefüllte Dualzemente sowie normalgefüllte fluoridhaltige Zahnlacke.

Als Bindemittel kommen für den Dentalwerkstoff alle diejenigen Bindemittel auf Basis eines polymerisierbaren, ethylenisch ungesättigten Monomeren in Frage, die dem Fachmann für diesen Einsatzzweck geläufig sind. Zu den mit Erfolg einsetzbaren polymerisierbaren Monomeren gehören vorzugsweise solche mit acrylischen und/oder methacrylischen Gruppen.

Insbesondere handelt es sich hierbei u. a. um Ester der α-Cyanoacrylsäure, (Meth)acrylsäure, Urethan(meth)acrylsäure, Crotonsäure, Zimtsäure, Sorbinsäure, Maleinsäure und Itaconsäure mit ein- oder zweiwertigen Alkoholen; (Meth)acrylamide wie z. B. N-isobutylacrylamid; Vinylester von Carbonsäuren wie z. B. Vinylacetat; Vinylether wie z. B. Butylvinylether; Mono-N-vinyl-Verbindungen wie N-Vinylpyrrolidon; und Styrol sowie seine Derivate. Besonders bevorzugt sind die nachfolgend aufgeführten mono- und polyfunktionellen (Meth)acrylsäureester und Urethan(meth)acrylsäureester.
(a) Monofunktionelle (Meth)acrylate
   Methyl(meth)acrylat, n- oder i-Propyl(meth)acrylat, n-, i- oder tert.-Butyl(meth)acrylat und 2-Hydroxyethyl(meth)acrylat.
(b) Difunktionelle (Meth)acrylate
   Verbindungen der allgemeinen Formel: 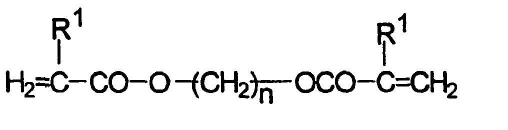 worin R¹ Wasserstoff oder Methyl ist und n eine positive ganze Zahl zwischen 3 und 20, wie z. B. Di(meth)acrylat des Propandiols, Butandiols, Hexandiols, Octandiols, Nonandiols, Decandiols und Eicosandiols,
   Verbindungen der allgemeinen Formel: 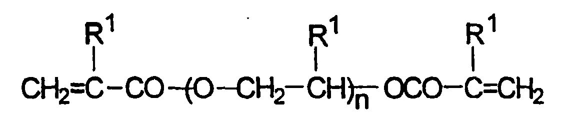 worin R¹ Wasserstoff oder Methyl ist und n eine positive ganze Zahl zwischen 1 und 14, wie z. B. Di(meth)acrylat des Ethylenglycols, Diethylenglycols, Triethylenglycols, Tetraethylenglycols, Dodecaethylenglycols, Tetradecaethylenglycols, Propylenglycols, Dipropylenglycols und Tetradecapropylenglycols; und Glycerindi(meth)acrylat, 2,2'-Bis[p-(γ-methacryloxy-β-hydroxypropoxy)-phenylpropan] oder Bis-GMA, Bisphenol-A-dimethacrylat, Neopentylglycoldi(meth)acrylat, 2,2'-Di(4-methacryloxypolyethoxyphenyl)propan mit 2 bis 10 Ethoxygruppen pro Molekül und 1,2-Bis(3-methacryloxy-2-hydroxypropoxy)butan.
(c) Tri- oder mehrfachfunktionelle (Meth)acrylate
   Trimethylolpropantri(meth)acrylat und Pentaerythritoltetra(meth)acrylat.
(d) Urethan(meth)acrylate
   Umsetzungsprodukte von 2 Mol hydroxylgruppenhaltigen (Meth)acrylatmonomer mit einem Mol Diisocyanat und Umsetzungsprodukte eines zwei NCO Endgruppen aufweisenden Urethanprepolymers mit einem methacrylischen Monomer, das eine Hydroxylgruppe aufweist, wie sie z. B. durch die allgemeine Formel wiedergegeben werden: 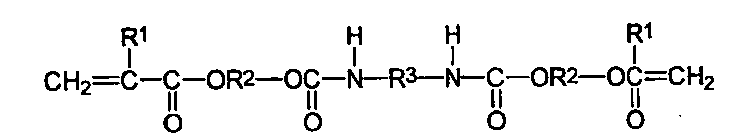 worin R¹ Wasserstoff oder eine Methylgruppe bedeutet, R² eine Alkylengruppe und R³ einen organischen Rest verkörpert.

Zu ganz besonders vorteilhaft im erfindungsgemäßen Dentalwerkstoff eingesetzten Monomeren gehören vor allem 2,2-Bis-4-(3-methacryloxy-2-hydroxypropoxy)-phenylpropan (Bis-GMA), 3,6-Dioxaoctamethylendimethacrylat (TEDMA), und/oder 7,7,9-Trimethyl-4,13-dioxo-3,14-dioxa-5,12-diazahexadecan-1,16-dioxy-dimethacrylat (UDMA).

Eine Beschreibung der Epoxide ist beispielsweise in DE 961 48 283 A1 gegeben.

Die Bezeichnung Ormocere beschreibt organisch modifizierte Polysiloxane wie sie beispielsweise in DE 41 33 494 C2 oder in DE 44 16 857 aufgeführt sind und für Dentalmassen verwendet werden können.

Flüssigkristalline Dentalmonomere sind in EP 0 754 675 A2 beschrieben.

Oxethane als Dentalmonomere sind in US 5 750 590 und DE 951 06 222 A1 beschrieben.

Spiroorthocarbonate werden beispielsweise in US 5 556 896 beschrieben.

Die genannten Monomeren werden entweder allein oder in Form einer Mischung von mehreren Monomeren verwendet.

Der Dentalwerkstoff kann je nach Art des verwendeten Katalysators heiß, kalt und/oder durch Licht polymerisierbar sein. Als Katalysatoren für die Heißpolymerisation können die bekannten Peroxide wie Dibenzoylperoxid, Dilauroylperoxid, tert.-Butylperoctoat oder tert.-Butylperbenzoat eingesetzt werden, aber auch α,α'-Azo-bis(isobutyroethylester), Benzpinakol und 2,2'-Dimethylbenzpinakol sind geeignet.

Als Katalysatoren für die Photopolymerisation können z. B. Benzophenon und seine Derivate sowie Benzoin und seine Derivate verwendet werden. Weitere bevorzugte Photosensibilisatoren sind α-Diketone wie 9,10-Phenanthrenchinon, Diacetyl, Furil, Anisil, 9,4'-Dichlorbenzile und 4,4'-Dialkoxybenzile, Campherchinon wird besonders bevorzugt verwendet. Die Verwendung der Photosensibilisatoren zusammen mit einem Reduktionsmittel wird bevorzugt. Beispiele für Reduktionsmittel sind Amine wie Cyanethylmethylanilin, Dimethylaminoethylmethacrylat, Triethylamin, Triethanolamin, N,N-Dimethylanilin, N-Methyldiphenylamin, N,N-Dimethyl-sym.-xylidin und N,N-3,5-Tetramethylanilin und 4-Dimethylaminobenzoesäureethylester.

Als Katalysatoren für die Kaltpolymerisation werden Radikale liefernde Systeme, z. B. Benzoyl- bzw. Lauroylperoxid zusammen mit Aminen wie N,N-Dimethyl-sym.-xylidin oder N,N-Dimethyl-p-toluidin verwendet. Es können auch dual härtende Systeme zur Katalyse verwendet werden, z. B. Photoinitiatoren mit Aminen und Peroxiden. Als Photokatalysatoren kommen auch Mischungen aus UV-lichthärtenden und im Bereich des sichtbaren Lichts härtenden Katalysatoren in Betracht.

Die Menge dieser Katalysatoren im Dentalwerkstoff liegt üblicherweise zwischen 0,01 bis 5 Gew.-%.

Vorzugsweise dient der erfindungsgemäße Dentalwerkstoff als Zahnfüllungsmaterial. Zahnfüllungsmaterialien werden auch als Zweikomponentenmaterialien hergestellt, die nach dem Anmischen kalt aushärten. Die Zusammensetzung ist ähnlich wie bei den lichthärtenden Materialien, nur wird anstatt der Photokatalysatoren in die eine Paste z. B. Benzoylperoxid und in die andere Paste z. B. N,N-Dimethyl-p-toluidin eingearbeitet. Durch Vermischen etwa gleicher Teile der beiden Pasten erhält man ein Zahnfüllungsmaterial, welches in wenigen Minuten aushärtet.

Wenn man bei den letztgenannten Materialien das Amin wegläßt und als Katalysator z. B. nur Benzoylperoxid verwendet, erhält man einen heißhärtenden Dentalwerkstoff, der für die Herstellung eines Inlays bzw. von künstlichen Zähnen verwendet werden kann. Für die Herstellung eines Inlays wird im Mund des Patienten von der Kavität ein Abdruck genommen und ein Gipsmodell hergestellt. In die Kavität des Gipsmodells wird die Paste eingebracht und das Ganze wird in einem Drucktopf unter Hitze polymerisiert. Das Inlay wird entnommen, bearbeitet und dann im Munde des Patienten in die Kavität einzementiert.

Unter einer Glaskeramik wird im Rahmen der Erfindung ein teilkristalliner Stoff verstanden, welcher aus amorphen SiO₂-Kompartimenten aufgebaut ist, in welche Kompartimente aus kristallinen erfindungsgemäßen Oxiden eingebettet vorhanden sind (siehe auch Ullmann's Encyclopedia of Industrial Chemistry 5^{th} Ed., A12, S.433ff).

Die Möglichkeit nach Art und Quantität im Rahmen der Erfindung die kristallinen erfindungsgemäßen Oxide, welche in den amorphen Glasverbund des Füllstoffs (A) eingebaut sind, frei auszuwählen, ist mithin ursächlich dafür, daß man den Brechungsindex des Füllstoffs (A) an den Brechungsindex der Polymerumgebung anpassen kann. Erst so wird gewährleistet, daß der Dentalwerkstoff als ganzes so transparent wird, daß er wie gefordert in einem Stück in der Kavität des Zahnes ausgehärtet werden kann. Eine mühsame sequentielle Auftragung und Aushärtung des Dentalmaterials kann entfallen. Bevorzugt für diesen Zweck einzusetzende Oxide sind TiO₂, ZrO₂, BaO und WO₃, ganz besonders bevorzugt wird ZrO₂ verwandt.

Ebenso wie zur Modifikation des Brechungsindex kann auch die Röntgenopazität des Füllstoffes (A) in idealer Weise durch Einbettung von kristallinen erfindungsgemäßen Oxidkomponenten in die amorphe Glasmatrix eingestellt werden. Bevorzugt für diesen Zweck einzusetzende Oxide sind TiO₂, ZrO₂, BaO, besonders bevorzugt wird ZrO₂ eingesetzt.

Als weitere Füllstoffe (B) kommen alle dem Fachmann geläufigen Füllstoffe zur Verbesserung der Qualität des Dentalwerkstoffs (Röntgenopazität, Viskosität, Polierbarkeit, Transparenz, Festigkeit, Brechungsindex) in Frage. Zur Erzielung einer weiter erhöhten Röntgenopazität können Füllstoffe eingesetzt werden, welche z. B. in der DE-OS 35 02 594 beschrieben sind, wobei deren mittlere Primärteilchengröße 5,0 µm nicht übersteigen sollte. Gegebenenfalls können geringe Mengen an mikrofeiner, pyrogener oder naßgefällter Kieselsäure als Füllstoff (B) in den Dentalwerkstoff eingearbeitet werden.

Als weitere Füllstoffe (B) kommen in Frage: Apatite gemäß der EP 0 832 636 und/oder Partikel gemäß der DE 195 08 586 und/oder DE 41 23 946.

Gegenstand der Erfindung ist auch ein Verfahren zur Herstellung des erfindungsgemäßen Dentalwerkstoffs. Dieses ist dadurch gekennzeichnet, daß man den Füllstoff (A) vor der Zubereitung des Dentalwerkstoffs mit gasförmigen oder flüssigen polymerisierbaren, ethylenisch ungesättigten Monomeren, Epoxiden, Ormoceren, flüssigkristallinen Monomeren, Oxethanen, Spiroorthoester bzw. -carbonaten belädt. Optional sind bei der Beladung die oben angegebenen Katalysatoren zugegen, die dann ebenfalls in den Poren des Füllstoffs (A) imbibiert vorliegen.

Anschließend kann der beladene Füllstoff (A) so in organische Matrix eingearbeitet werden oder die in dem Füllstoff (A) vor der Zubereitung des Dentalwerkstoffs befindlichen polymerisierbaren, ethylenisch ungesättigten Monomere werden vor der Einarbeitung in deren polymerisierte Form überführt.

Bevorzugt ist allerdings die Ausführungsform, bei der man die in dem Füllstoff (A) vor der Zubereitung des Dentalwerkstoffs befindlichen polymerisierbaren, ethylenisch ungesättigten Monomere in deren polymerisierte Form überführt und anschließend die Einarbeitung in die organische Matrix vornimmt.

Zusätzlich kann der Füllstoff (A) vor der Einarbeitung in die Matrix zusätzlich mit bacteriziden Mitteln beladen werden oder chemisch modifiziert werden. Es gilt diesbezüglich das weiter oben zu diesem Punkt Ausgeführte.

Das Beladen des Füllstoffs (A) erfolgt also bevorzugt durch Behandlung des mikro- und/oder mesoporösen glaskeramischen Füllstoffs (A) mit einem Dampf oder Gas sowie alternativ mit flüssigen polymerisierbaren, ethylenisch ungesättigten oben beschriebenen Monomeren und ggf. den ebenfalls oben beschriebenen Katalysatoren. Arbeitet man den beladenen Füllstoff jetzt in die organische Matrix ein, so kann sich bei der folgenden Polymerisation das Monomer in den Poren mit dem außerhalb des Füllstoffs (A) befindlichen Monomer zu einem Durchdringungsgefüge verbinden. Dieser Vorgang erfolgt ebenfalls bei der oben als bevorzugt beschilderten sequentiellen Polymerisation von Beladungsmaterial und Matrix. Durch das sich im Füllstoff (A) ausgebildete polymere Netzwerk, welches exoseitig mit dem Bindemittel des Dentalwerkstoffs chemisch verbunden ist, wird gewährleistet, daß sich eine überaus starke physikalische Bindung zwischen dem Füllstoff (A) und dem Bindemittel des Dentalwerkstoffs ausbildet, welche durch Hydrolyse nicht mehr zerstört werden kann. Gleichsam erhält man einen Dentalwerkstoff, der sich durch eine besondere Abrasionsfestigkeit bei gleichzeitig hoher Biegefestigkeit auszeichnet, die von vergleichbaren Dentalwerkstoffen des Standes der Technik nicht erreicht wird. Auch wird damit die Spaltung zwischen den organischen Partikeln und der organischen Matrix fast gänzlich unterdrückt. Dies steigert die Haltbarkeit der erfindungsgemäßen Dentalmassen, da eine mit der Zeit einsetzende hydrolytische Spaltung der Bindung zwischen organischen und anorganischen Bestandteilen wie von den Materialien des Standes der Technik bekannt nicht mehr auftreten kann.

Ein weiterer Aspekt der Erfindung beschäftigt sich mit porösen Glaskeramiken, welche mit polymerisierbaren, ethylenisch ungesättigten Monomeren oder mit deren polymerisierter Form beladen sind. Durch deren Bereitstellung gelingt es, in einem preiswerten Verfahren Füllstoffe für Dentalwerkstoffe zur Verfügung zu stellen, welche den Materialien des Standes der Technik überlegen sind. Bevorzugt setzt man für diesen Zweck poröse Glaskeramiken ein, welche eine Porengröße von > 1 nm und < 1000 nm aufweisen, deren Partikelgröße zwischen 10 und 30 µm beträgt und die ein Porenvolumen von > 500 mm³/g und < 1500 mm³/g besitzen. Der kristalline Anteil der Glaskeramik besteht bevorzugt aus Oxiden der Metalle der 1.-4. Hauptgruppe und/oder aus Oxiden der Metalle der Nebengruppen. Diese Oxide sind in der Glaskeramik zu einem Anteil von 1-50 Gew.-%, bezogen auf die Glaskeramik, enthalten. Ansonsten gilt bezüglich der Glaskeramik das für den Füllstoff (A) Gesagte.

In einem nächsten Aspekt betrifft die Erfindung eine poröse Glaskeramik, deren Porenvolumen zwischen 500 mm³/g und 1500 mm³/g beträgt. Erfindungsgemäß bevorzugt weist diese eine Porengröße von > 1 nm und < 1000 nm auf. Die Partikelgröße der Keramik kann bevorzugt zwischen 10 und 30 µm liegen. Sie ist neben ihrem amorphen SiO₂-Anteil aus kristallinen Bereichen aufgebaut, die aus Oxiden der Metalle der 1.-4. Hauptgruppe sowie aus Oxiden der Nebengruppenmetalle bestehen. Der Anteil dieser Oxide am glaskeramischen Körper beträgt zwischen 1 und 50 Gew.-%. Ansonsten gilt bezüglich der porösen Glaskeramik das für die beladene Glaskeramik Gesagte.

Ein weiterer Aspekt der Erfindung richtet sich auf ein erfindungsgemäßes Verfahren zur Herstellung der Glaskeramiken, bei dem man die Glaskeramik durch Vermischen eines wäßrigen SiO₂-Sols und einer oder mehrerer wäßriger Sole von Oxiden der Metalle der 1. bis 4. Hauptgruppe sowie von Oxiden der Metalle der Nebengruppen herstellt.

Bevorzugt kann man dem Solgemisch zusätzlich 0-20 Gew.-%, bezogen auf das Solgemisch, einer wäßrigen Lösung eines Salzes hinzufügen. Als Salze können im Prinzip alle dem Fachmann für diesen Zweck in Frage kommenden Metallsalze verwendet werden. Bevorzugt sind Metallsalze wie Alkalinitrate und/oder -acetate sowie Nitrate der Metalle der Nebengruppen einzusetzen. Die Zugabe der Lösungen von Metallsalzen zur Solmischung ermöglicht die Herstellung von Pulvern mit hoher Festigkeit. Salze beeinflussen aber auch die Oberflächenchemie der resultierenden Pulver, was für viele Verwendüngszwecke (Katalysatoren) von entscheidender Bedeutung ist. Außerdem bestimmen sie in hohem Maße das Verhalten der Pulver während der nachfolgenden Temperaturbehandlung. Es ist so beispielsweise möglich Teilchen aufzubauen, welche einen chemischen Gradienten der Zusammensetzung von Innen nach Außen aufweisen und so Keramikpulverkörner entstehen, bei denen Hülle und Kern andere chemische und physikalische Eigenschaften aufweisen.

Ebenso kann der Solmischung ggf. 0-10 Gew.-% (bezogen auf das Gemisch) an organischen Lösungsmitteln zugefügt werden. Als bevorzugt einzusetzende organische Lösungsmittel sind kurzkettige Alkohole, kurzkettige Säuren und chelatbildende Lösungsmittel wie Acetylacetonat zu nennen.

Anschließend wird das Solgemisch im Gasstrom getrocknet. Bevorzugt wird das Solgemisch sprühgetrocknet. Im Prinzip können jedoch auch andere, dem Fachmann bekannten Trucknungsprozedere wie beispielsweise die Lyophilisation zur Herstellung der Glaskeramik dienen.

Abschließend wird sprühgetrocknetes Solgemisch bei > 800°C und < 1300°C calciniert.

Die beladene oder unbeladene poröse Glaskeramik kommt bevorzugt in Dentalwerkstoffen zum Einsatz.

Die Herstellung von Keramikpulvern mit den oben genannten Eigenschaften erfordert die Verwendung unterschiedlicher Precursoren, die jeweils ein Hauptmerkmal des resultierenden Pulvers dominant beeinflussen.

Als Precursoren werden ein Oxid des Siliziums, mindestens ein weiteres Oxid der Metalle der 1. bis 4. Hauptgruppe oder der Nebengruppenmetalle und eventuell ein Salz gewählt.

Als Siliziumoxide eignen sich z.B. auf pyrolytischem Wege hergestellte Pulver. Sie werden in einem wäßrigen Lösungsmittel suspendiert, so daß ein Sol entsteht. Bei geeigneter Wahl der Ausgangssole entstehen Granulate, deren Verhältnis der Porenweiten d₁₀ und d₉₀ die Zahl 2,5 nicht übersteigt. d₁₀ bezeichnet dabei die Porenweite, die der Anteil von 10 Vol-% der größten Poren nicht unterschreitet, und d₉₀ bezeichnet die Porenweite, die der Anteil von 90 Vol-% der größten Poren nicht unterschreitet.

Als Oxide, die für die Einstellung der physikalischen und chemischen Eigenschaften verantwortlich sind, können alle Oxide verwendet werden, die als Suspension nicht agglomerierter, nanoskaliger Teilchen hergestellt werden können. Hier eignen sich vor allem Sole, die durch Hydrolyse- und Kondensationsreaktionen von Metallsalzen und -alkoholaten entstehen.

Lösungen von Metallsalzen können als dritte Komponente zugegeben werden. Sie ermöglichen die Herstellung von Glaskeramiken mit hoher Festigkeit.

Die Keramikpulver werden hergestellt, indem das wäßrige Sol eines Oxids des Siliziums mit definiert agglomerierten Primärteilchen, das keinen oder nur einen geringen Anteil organischer Zusätze enthält, mit dem oder den Solen andere Oxide und den Salzlösungen vermischt und dann im heißen Gasstrom durch Lösungsmittelentzug verfestigt wird. Hierfür eignet sich der Sprühtrocknungsprozeß. Mit Hilfe einer nachfolgenden Temperaturbehandlung können agglomeratfreie Keramikpulver mit kugelförmigen Körnern, einstellbarem Porenvolumen und hoher Festigkeit hergestellt werden.

Die hierin vorgestellten erfindungsgemäßen Dentalwerkstoffe sind aufgrund der preiswert herzustellenden und dennoch nicht minder vorteilhaften glaskeramischen Füllstoffe (A) in ökonomisch akzeptabler Weise zu bereiten.

Durch das Auffinden der neuen porösen Glaskeramiken bzw. Füllstoffe (A) als Komponenten in neuen Dentalwerkstoffen erhält man mithin annehmbare (im Hinblick auf Bearbeitbarkeit, modische Akzeptanz, Festigkeit, Abrasionsfestigkeit, Polymerschrumpf, Haltbarkeit, Preis etc.) Alternativen gegenüber den im Kaubereich immer noch größtenteils eingesetzten Amalgamen.

Die folgenden Beispiele sollen die Erfindung erläutern.

### Beispiel 1

Mit Hilfe eines Ultraturrax wird AEROSIL® 200 (Degussa AG) in Wasser eingearbeitet, so daß eine Suspension entsteht. Dieses Sol wird 2 Tage auf einem Schüttler bewegt. Das SiO₂-Sol wird danach mit den unten genannten Solen vermischt, so daß die in der Tabelle genannten Masseverhältnisse der Feststoffe resultieren.

Ein Zirkonoxidsol wird hergestellt, indem man eine essigsaure Zirkoniumacetatlösung erwärmt, bis die Gelierung eintritt. Durch geeignete Wahl des pH-Wertes und Temperaturerniedrigung läßt sich das Gel wieder peptisieren, so daß ein Sol mit isolierten ZrO₂-Teilchen entsteht, deren Durchmesser kleiner als 30 nm sind. Das Sol ist transparent. Der pH-Wert muß so eingestellt werden, daß beim Vermischen mit dem SiO₂-Sol keine spontane Gelierung eintritt.

Ein Titanoxidsol wird hergestellt, indem man eine basische wäßrige Lösung von Titan(IV)-bis-(ammoniumlactat)dihydroxid erwärmt, bis erste Opaleszenz auftritt. Das Sol bleibt auch bei der Lagerung weitgehend transparent. Der pH-Wert muß so eingestellt werden, daß beim Vermischen mit dem SiO₂-Sol keine spontane Gelierung eintritt.

Durch Sprühtrocknung der Solmischungen und anschließender Calcinierung lassen sich Pulver der unten genannten Zusammensetzungen herstellen. Pulver, die ausschließlich mit Hilfe des SiO₂-Sols hergestellt wurden, sind zum Vergleich mit aufgenommen.

Die Sprühtrocknung erfolgt bei Temperaturen kleiner 200 °C bei konstantem Durchfluß.

| Masseanteil ZrO₂ | Masseanteil SiO₂ | Brechungsindex n_{D} | Dichte (g/cm³) |
|---|---|---|---|
| 0 | 100 | 1,472(2) | 2,26(1) |
| 10 | 90 | 1,509(2) | 2,39(1) |
| 30 | 70 | 1,59(1) | 2,70(1) |
| 50 | 50 | 1,68(1) | 3,23(1) |

### Beispiel 2

Die Herstellung der unten genannten Pulver entspricht weitgehend der in Beispiel 1 genannten, es wird jedoch zur Herstellung des SiO₂-Sols zum einen AEROSIL^{®} 200 (Degussa AG) und zum anderen AEROSIL^{®} 90 (Degussa AG) verwendet. Die Porenweiten, die durch das verwendete AEROSIL^{®} bestimmt werden, bleiben auch bei Zusatz von ZrO₂ erhalten.

| Masseanteil ZrO₂ | Masseanteil SiO₂ | AEROSIL^{®}-Art | Calcinierungs-temperatur (°C) | Porenweite (nm) |
|---|---|---|---|---|
| 0 | 100 | AEROSIL^{®} 90 | 1100 | 62(2) |
| 10 | 90 | AEROSIL^{®} 90 | 1100 | 60(2) |
| 0 | 100 | AEROSIL^{®} 200 | 1050 | 32(2) |
| 10 | 90 | AEROSIL^{®} 200 | 1050 | 32(2) |

### Beispiel 3

Durch eine Variation der Calcinierungstemperatur bzw. der Calcinierungszeit läßt sich das Porenvolumen über einen großen Bereich bei weitestgehender Konstanz der Porenweiten variieren, wie aus der untenstehenden Tabelle beispielhaft für ein Pulver mit 10 Gew.-% ZrO₂ zu entnehmen ist. Eine Variation der Calcinierungszeit führt zu einem ähnlichen Verhalten.

| Calcinierungstemperatur (°C) | Calcinierungszeit (h) | Porenvolumen (mm³/g) | Porenweite (nm) | Spez. Oberfläche (m²/g) |
|---|---|---|---|---|
| 900 | 5 | n.b. | 35(2) | n.b. |
| 1000 | 5 | 1279(64) | 33(2) | 125 |
| 1050 | 5 | 881(44) | 32(2) | 115 |
| 1100 | 5 | 287(15) | 25(2) | 52 |

### Beispiel 4

Durch die Verwendung von Metallsalzen zusätzlich zu den in Beispiel 2 genannten Ausgangsstoffen kann beispielsweise die Calcinierung gesteuert werden. Ein Beispiel für die Porencharakteristik, wie sie für eine Glaskeramik mit 10 % ZrO₂ und 90 % SiO₂ bei Zusatz von Natriumnitrat zu beobachten ist, liefert die untenstehende Tabelle.

| Zusatz von Natriumnitrat | Calcinierungstemperatur (°C) | Calcinierungszeit (h) | Porenvolumen (mm³/g) | Porenweite (nm) | Spez. Oberfläche (m²/g) |
|---|---|---|---|---|---|
| nein | 900 | 5 | n.b. | 35(2) | n.b. |
| nein | 1000 | 5 | 1279(64) | 33(2) | 125 |
| ja | 900 | 5 | n.b. | 31(2) | n.b. |
| ja | 1000 | 5 | 172 | 20(2) | 40 |

Die beschriebenen porösen Füllstoffe werden zu Dental-Füllungswerkstoffen weiterverarbeitet. Dazu werden diese mit weiteren Komponenten vermischt, wie in Beispiel 5 beschrieben. Die Bestimmung der Biegefestigkeit und des E-Moduls erfolgt im 3-Punkt-Biegeversuch. Einzelheiten zur Probengeometrie und dem Prüfverfahren sind der Norm ISO 4049:1988 zu entnehmen.

### Beispiel 5:

In 47,13 g einer Monomermischung, bestehend aus 35 Teilen Bis-GMA, 35 Teilen UDMA und 30 Teilen TEGDMA, werden 52,87 g des silanisierten porösen Füllstoffes aus Beispiel 2 zusammen mit 0,034 Gew.-% Campherchinon unter Vacuum von 200 mbar eingearbeitet. Die entstehende Paste wird mit Licht (Degulux^{®}) ausgehärtet. Biegefestigkeit: 92 ± 6 MPa, E-Modul: 4765 ± 89 MPa.

### Vergleichsbeispiel Dentalwerkstoff:

Der Vergleich zum Stand der Technik bezieht sich auf das Produkt Solitaire der Fa. Kulzer. Dieses Material enthält ca. 30 Gew.-% eines porösen Füllstoffes, bestehend aus 100 % SiO₂ sowie zusätzlich zur Verstärkung noch ca. 30 Gew.-% kompakter Glaspartikel einer mittleren Partikelgröße von 0,7 µm. Die Messung der Festigkeitseigenschaften erfolgte in oben beschriebener Art und Weise. Biegefestigkeit: 51 ± 6 MPa, E-Modul: 2765 ± 89 MPa.

## Patentansprüche

1. Dentalwerkstoff auf Basis von polymerisierbaren, beispielsweise ethylenisch ungesättigten Monomeren, Epoxiden, Ormoceren, flüssigkristallinen Monomeren, Oxethanen, Spiroorthoester bzw. -carbonaten als Bindemittel, eines Katalysators für die Kalt-, Heiß- und/oder Photopolymerisation und bezogen auf den Dentalwerkstoff 20-70 Gew.-% eines anorganischen Füllstoffs (A) sowie bezogen auf den Dentalwerkstoff 0-60 Gew.-% von weiteren Füllstoffen (B) sowie 0-2 Gew.-% von weiteren üblichen Zuschlagstoffen,
**dadurch gekennzeichnet,**
**dass** der anorganische Füllstoff (A) eine poröse Glaskeramik ist, deren Mikro-und/oder Mesoporen mit polymerisierbaren, ethylenisch ungesättigten Monomeren, Epoxiden, Ormoceren, flüssigkristallinen Monomeren, Oxethanen, Spiroorthoester bzw. -carbonaten oder mit deren polymerisierter Form beladen sind und der anorganische Füllstoff (A) zusätzlich ein kristallines Oxid mit einer Konzentration aufweist, dass der Brechungsindex des Füllstoffs (A) an den Brechungsindex der Polymerumgebung anpassbar ist und der anorganische Füllstoff (A) ein Porenvolumen größer als 200 und kleiner als 2000 mm³/g aufweist.

2. Dentalwerkstoff nach Anspruch 1,
**dadurch gekennzeichnet,**
**daß** die Porengröße des Füllstoffs (A) größer als 10 nm und kleiner als 100 nm ist.

3. Dentalwerkstoff nach Anspruch 1 und/oder 2,
**dadurch gekennzeichnet,**
**daß** die Partikelgröße des Füllstoffs (A) zwischen 10 und 30 µm beträgt.

4. Dentalwerkstoff nach einem oder mehreren der vorhergehenden Ansprüche,
**dadurch gekennzeichnet,**
**daß** das Porenvolumen des Füllstoffs (A) größer als 500 mm³/g und kleiner als 1500 mm³/g ist.

5. Dentalwerkstoff nach einem oder mehreren der vorhergehenden Ansprüche,
**dadurch gekennzeichnet,**
**daß** der Füllstoff (A) Oxide der Metalle der 1. bis 4. Hauptgruppe sowie Oxide der Metalle der Nebengruppen enthält.

6. Dentalwerkstoff nach Anspruch 5,
**dadurch gekennzeichnet,**
**dass** das kristalline Oxid ausgewählt ist aus der Gruppe, bestehend aus TiO₂, ZrO₂, BaO, WO₃ und Fe₂O₃.

7. Dentalwerkstoff nach Anspruch 5-6,
**dadurch gekennzeichnet,**
**daß** die Oxide einzeln oder im Gemisch zwischen 1 und 50 Gew.-% im Füllstoff (A) vorhanden sind.

8. Dentalwerkstoff nach einem oder mehreren der vorhergehenden Ansprüche,
**dadurch gekennzeichnet, daß** im auspolymerisierten Zustand das Verhältnis der Gew.-Teile von Polymer zu Füllstoff (A) zu Füllstoffen (B) bei Dentalfüllungen im Verhältnisbereich von 30-50 zu 30-60 zu 5-20 liegt (in Summe 100).

9. Dentalwerkstoff nach einem oder mehreren der vorhergehenden Ansprüche,
**dadurch gekennzeichnet,**
**daß** der Füllstoff (A) zusätzlich mit bacteriziden Mitteln beladen ist.

10. Dentalwerkstoff nach einem oder mehreren der vorhergehenden Ansprüche,
**dadurch gekennzeichnet,**
**daß** die Oberfläche des Füllstoffs (A) und/oder (B) chemisch modifiziert ist.

11. Verfahren zur Herstellung eines Dentalwerkstoffs nach Anspruch 1,
**dadurch gekennzeichnet,**
**daß** man den Füllstoff (A) vor der Zubereitung des Dentalwerkstoffs mit gasförmigen polymerisierbaren, ethylenisch ungesättigten Monomeren belädt.

12. Verfahren zur Herstellung eines Dentalwerkstoffes nach Anspruch 1,
**dadurch gekennzeichnet,**
**daß** man den Füllstoff (A) vor der Zubereitung des Dentalwerkstoffs mit flüssigen polymerisierbaren, ethylenisch ungesättigten Monomeren belädt.

13. Verfahren nach einem oder mehreren der Ansprüche 11-12,
**dadurch gekennzeichnet,**
**daß** man die in dem Füllstoff (A) vor der Zubereitung des Dentalwerkstoffs befindlichen polymerisierbaren, ethylenisch ungesättigten Monomere in deren polymerisierte Form überführt.

14. Verfahren nach einem oder mehreren der Ansprüche 11-13,
**dadurch gekennzeichnet,**
**daß** man den Füllstoff (A) zusätzlich mit bacteriziden Mitteln belädt.

15. Verfahren nach einem oder mehreren der Ansprüche 11-14,
**dadurch gekennzeichnet,**
**daß** man die Oberfläche des Füllstoffs (A) und/oder (B) chemisch modifiziert.

16. Poröse Glaskeramik, deren Mikro- und/oder Mesoporen mit polymerisierbaren, ethylenisch ungesättigten Monomeren, Epoxiden, Ormoceren, flüssigkristallinen Monomeren, Oxethanen, Spiroorthoester bzw. -carbonaten oder mit deren polymerisierter Form beladen sind, die zusätzlich ein kristallines Oxid mit einer Konzentration aufweist, dass der Brechungsindex des Füllstoffs (A) an den Brechungsindex der Polymerumgebung anpassbar ist und wobei die poröse Glaskeramik ein Porenvolumen größer als 200 und kleiner als 2000 mm³/g aufweist.

17. Poröse Glaskeramik nach Anspruch 16,
**dadurch gekennzeichnet,**
**daß** die Porengröße der Glaskeramik größer als 1 nm und kleiner als 1000 nm ist.

18. Poröse Glaskeramik nach Anspruch 16 und/oder 17,
**dadurch gekennzeichnet,**
**daß** die Partikelgröße der Glaskeramik zwischen 10 und 30 µm beträgt.

19. Poröse Glaskeramik nach einem oder mehreren der Ansprüche 16 bis 18,
**dadurch gekennzeichnet,**
**daß** das Porenvolumen der Glaskeramik größer als 150 mm³/g und kleiner als 1500 mm³/g ist.

20. Poröse Glaskeramik nach einem oder mehreren der Ansprüche 16 bis 19,
**dadurch gekennzeichnet,**
**daß** die Glaskeramik Oxide der Metalle der 1. bis 4. Hauptgruppe sowie Oxide der Metalle der Nebengruppen enthält.

21. Poröse Glaskeramik nach Anspruch 20,
**dadurch gekennzeichnet,**
**dass** das kristalline Oxid ausgewählt ist aus der Gruppe, bestehend aus TiO₂, ZrO₂, BaO, WO₃ und Fe₂O₃.

22. Poröse Glaskeramik nach Anspruch 20-21,
**dadurch gekennzeichnet,**
**daß** die Oxide einzeln oder im Gemisch zwischen 1 und 50 Gew.-% in der Glaskeramik vorhanden sind.

23. Verfahren zur Herstellung der porösen Glaskeramik nach Anspruch 16,
**dadurch gekennzeichnet,**
**daß** man die Glaskeramik durch Vermischen eines wäßrigen SiO₂-Sols und eines oder mehrerer Sole von Oxiden der Metalle der 1. bis 4. Hauptgruppe sowie von Oxiden der Metalle der Nebengruppen herstellt.

24. Verfahren nach Anspruch 23,
**dadurch gekennzeichnet,**
**daß** man dem Gesamtsol eine wäßrige Lösung eines Salzes hinzufügt.

25. Verfahren nach Anspruch 23 und/oder 24,
**dadurch gekennzeichnet,**
**daß** man dem Solgemisch 0-10 Gew.% (bezogen auf das Solgemisch) an organischen Lösungsmitteln zufügt.

26. Verfahren nach einem oder mehreren der Ansprüche 23 bis 25.
**dadurch gekennzeichnet,**
**daß** man das Solgemisch sprühtrocknet.

27. Verfahren nach Anspruch 26,
**dadurch gekennzeichnet,**
**daß** man das sprühgetrocknete Solgemisch bei > 800°C und < 1300°C calciniert.

28. Verwendung der porösen Glaskeramiken nach Anspruch 16 in Dentalwerkstoffen nach Anspruch 1.

29. Verwendung der porösen Glaskeramik nach Anspruch 16 zum Beladen mit polymerisierbaren, ethylenisch ungesättigten Verbindungen.

## Claims

1. Dental material based on polymerizable, for example ethylenically unsaturated monomers, epoxides, ormocers, liquid crystalline monomers, oxethanes, spiro-orthoesters or -carbonates as binders, a catalyst for cold, hot and/or photopolymerization and, based on the dental material, 20-70 wt.% of an inorganic filler (A) and, based on the dental material, 0-60 wt.% of further fillers (B) and 0-2wt.% of other conventional additives,
**characterized in**
**that** the inorganic filler (A) is a porous glass ceramic in which the micro- and/or mesopores are charged with polymerizable, ethylenically unsaturated monomers, epoxides, ormocers, liquid crystalline monomers, oxethanes, spiro-orthoesters or - carbonates or with the polymerized form thereof, and that the inorganic filler (A) comprises additionally a crystalline oxide with a concentration that the refraction index of the filler (A) is adaptable to the refraction index of the surrounding polymer and the inorganic filler (A) has a pore volume greater than 200 and less than 2000 mm³/g.

2. Dental material according to claim 1,
**characterized in**
**that** the pore size of filler (A) is greater than 10 nm and less than 100 nm.

3. Dental material according to claim 1 and/or 2,
**characterized in**
**that** the particle size of filler (A) is between 10 and 30 µm.

4. Dental material according to one or more of the preceding claims,
**characterized in**
**that** the pore volume of filler (A) is greater than 500 mm³/g and less than 1500 mm³/g.

5. Dental material according to one or more of the preceding claims,
**characterized in**
**that** the filler (A) comprises oxides of the metals of the main groups 1 to 4 and oxides of the metals of the sub-groups.

6. Dental material according to claim 5,
**characterized in**
**that** crystalline oxide is selected from the group consisting of TiO₂, ZrO₂, BaO, WO₃ and Fe₂O₃.

7. Dental material according to claim 5-6,
**characterized in**
**that** the oxides are present in filler (A) individually or as a mixture in an amount of between 1 and 50 wt.%.

8. Dental material according to one or more of the preceding claims,
**characterized in**
**that** in the polymerized state, the ratio of the parts by wt. of polymer to filler (A) to fillers (B) in dental fillings is in the ratio range of 30-50 to 30-60 to 5-20 (100 in total).

9. Dental material according to one or more of the preceding claims,
**characterized in**
**that** filler (A) is additionally charged with bactericidal agents.

10. Dental material according to one or more of the preceding claims,
**characterized in**
**that** the surface of filler (A) and/or (B) is chemically modified.

11. Process for the preparation of a dental material according to claim 1,
**characterized in**
**that** filler (A) is charged with gaseous polymerizable ethylenically unsaturated monomers before formulation of the dental material.

12. Process for the preparation of a dental material according to claim 1,
**characterized in**
**that** filler (A) is charged with liquid polymerizable, ethylenically unsaturated monomers before formulation of the dental material.

13. Process according to one or more of claims 11-12,
**characterized in**
**that** the polymerizable, ethylenically unsaturated monomers present in filler (A) before formulation of the dental material are converted into their polymerized form.

14. Process according to one or more of claims 11-13,
**characterized in**
**that** filler (A) is additionally charged with bactericidal agents.

15. Process according to one or more of claims 11-14,
**characterized in**
**that** the surface of filler (A) and/or (B) is chemically modified.

16. Porous glass ceramic in which the micro- and/or mesopores are charged with polymerizable, ethylenically unsaturated monomers, epoxides, ormocers, liquid crystalline monomers, oxethanes, spiro-orthoesters or -carbonates or with the polymerized form thereof, which comprises additionally a crystalline oxide with a concentration that the refraction index of the filler (A) is adaptable to the refraction index of the surrounding polymer and the inorganic filler (A) has a pore volume greater than 200 and less than 2000 mm³/g.

17. Porous glass ceramic according to claim 16,
**characterized in**
**that** the pore size of the glass ceramic is greater than 1 nm and less than 1000 nm.

18. Porous glass ceramic according to claim 16 and/or 17,
**characterized in**
**that** the particle size of the glass ceramic is between 10 and 30 µm.

19. Porous glass ceramic according to one or more of claims 16 to 18,
**characterized in**
**that** the pore volume of the glass ceramic is greater then 150 mm³/g and less than 1500 mm³/g.

20. Porous glass ceramic according to one or more of claims 16 to 19,
**characterized in**
**that** the glass ceramic comprises oxides of metals of the main groups 1 to 4 and oxides of metals of the sub-groups.

21. Porous glass ceramic according to claim 20,
**characterized in**
**that** the crystalline oxide is selected from the group consisting of TiO₂, ZrO₂, BaO, WO₃, and Fe₂O₃.

22. Porous glass ceramic according to claim 20 to 21,
**characterized in**
**that** the oxides are present in the glass ceramic individually or as a mixture in an amount of between 1 and 50 wt.%.

23. Process for the preparation of the porous glass ceramic according to claim 16,
**characterized in**
**that** the glass ceramic is prepared by mixing an aqueous SiO₂ sol and one or more sols of oxides of the metals of the main groups 1 to 4 and of oxides of the metals of the sub-groups.

24. Process according to claim 23,
**characterized in**
**that** an aqueous solution of a salt is added to the total sol.

25. Process according to claim 23 and/or 24,
**characterized in**
**that** 0-10 wt.% (based on the sol mixture) of organic solvents is added to the sol mixture.

26. Process according to one or more of claims 23 to 25,
**characterized in**
**that** the sol mixture is spray-dried.

27. Process according to claim 26,
**characterized in**
**that** the spray-dried sol mixture is calcined at > 800 °C and < 1300 °C.

28. Use of the porous glass ceramics according to claim 16 in dental materials according to claim 1.

29. Use of the porous glass ceramics according to claim 16 for charging with polymerizable, ethylenically unsaturated compounds.

## Revendications

1. Matériau dentaire à base de monomères, époxydes, ormocères, monomères liquides cristallins, oxétanes, spiroorthoesters ou spiroorthocarbonates polymérisables, par exemple éthyléniquement insaturés en tant que liants, d'un catalyseur pour la polymérisation à froid, à chaud et/ou la photopolymérisation et, par rapport au matériau dentaire, de 20 à 70 % en poids d'une charge inorganique (A) ainsi que, par rapport au matériau dentaire, de 0 à 60 % en poids d'autres charges (B) ainsi que de 0 à 2 % en poids d'autres additifs usuels,
**caractérisé en ce que**
la charge inorganique (A) est une vitrocéramique poreuse dont les micropores et/ou les mésopores sont chargés en monomères, époxydes, ormocères, monomères liquides cristallins, oxétanes, spiroorthoesters ou spiroorthocarbonates polymérisables, éthyléniques insaturés, ou en leur forme polymérisable et la charge inorganique (A) présente en plus un oxyde cristallin concentré, l'indice de réfraction de la charge (A) peut être adapté à l'indice de réfraction de l'environnement polymère et la charge inorganique (A) présente un volume poreux supérieur à 200 et inférieur à 2000 mm³/g.

2. Matériau dentaire selon la revendication 1,
**caractérisé en ce que**
la taille des pores de la charge (A) est supérieure à 10 nm et inférieure à 100 nm.

3. Matériau dentaire selon la revendication 1 et/ ou 2,
**caractérisé en ce que**
la taille des particules de la charge (A) est comprise entre 10 et 30 µm.

4. Matériau dentaire selon l'une quelconque ou plusieurs des revendications précédentes,
**caractérisé en ce que**
le volume poreux de la charge (A) est supérieur à 500 mm³/g et inférieur à 1500 mm³/g.

5. Matériau dentaire selon l'une quelconque ou plusieurs des revendications précédentes,
**caractérisé en ce que**
la charge (A) contient des oxydes des métaux du 1^{er} au 4^{ème} groupe principal ainsi que des oxydes des métaux des groupes secondaires.

6. Matériau dentaire selon la revendication 5,
**caractérisé en ce qu'**
on choisit l'oxyde cristallin dans le groupe constitué de TiO₂, ZrO₂, BaO, WO₃ et Fe₂O₃.

7. Matériau dentaire selon la revendication 5-6,
**caractérisé en ce que**
les oxydes sont présents individuellement ou en mélange entre 1 et 50 % en poids dans la charge (A).

8. Matériau dentaire selon l'une quelconque ou plusieurs des revendications précédentes,
**caractérisé en ce qu'**
en l'état polymérisé, le rapport des parties en poids de polymère à la charge (A) aux charges (B), dans les amalgames dentaires, est de l'ordre de 30-50 à 30-60 à 5-20 (au total 100).

9. Matériau dentaire selon l'une quelconque ou plusieurs des revendications précédentes,
**caractérisé en ce que**
la charge (A) est chargée, en plus, en produits bactéricides.

10. Matériau dentaire selon l'une quelconque ou plusieurs des revendications précédentes,
**caractérisé en ce que**
la surface de la charge (A) et/ ou (B) est modifiée chimiquement.

11. Procédé de fabrication d'un matériau dentaire selon la revendication 1,
**caractérisé en ce qu'**
on charge la charge (A), avant la préparation du matériau dentaire, en monomères gazeux polymérisables, éthyléniquement insaturés.

12. Procédé de fabrication d'un matériau dentaire selon la revendication 1,
**caractérisé en ce qu'**
on charge la charge (A), avant la préparation du matériau dentaire, en monomères liquides polymérisables, éthyléniquement insaturés.

13. Procédé de fabrication d'un matériau dentaire selon l'une quelconque ou plusieurs des revendications 11-12,
**caractérisé en ce qu'**
on transfère les monomères polymérisables, éthyléniquement insaturés se trouvant dans la charge (A), avant la préparation du matériau dentaire, en leur forme polymérisable.

14. Procédé de fabrication d'un matériau dentaire selon l'une quelconque ou plusieurs des revendications 11-13,
**caractérisé en ce qu'**
on charge la charge (A) en plus en produits bactéricides.

15. Procédé de fabrication d'un matériau dentaire selon l'une quelconque ou plusieurs des revendications 11-14,
**caractérisé en ce qu'**
on modifie chimiquement la surface de la charge (A) et/ ou (B).

16. Vitrocéramique poreuse, dont les micro et/ou mésopores sont chargés en monomères, époxydes, ormocères, monomères liquides cristallins, oxétanes, spiroorthoesters ou spiroorthocarbonates polymérisables, éthyléniquement insaturés ou en leur forme polymérisable qui présente en plus un oxyde cristallin concentré, l'indice de réfraction de la charge (A) peut être adapté à l'indice de réfraction de l'environnement polymère et la vitrocéramique poreuse présentant un volume poreux supérieur à 200 et inférieur à 2000 mm³/g.

17. Vitrocéramique poreuse selon la revendication 16,
**caractérisée en ce que**
la taille des pores de la vitrocéramique est supérieure à 1 nm et inférieure à 1000 nm.

18. Vitrocéramique selon la revendication 16 et/ou 17
**caractérisée en ce que**
la taille des particules de la vitrocéramique est comprise entre 10 et 30 µm.

19. Vitrocéramique poreuse selon l'une quelconque ou plusieurs des revendications 16 à 18,
**caractérisée en ce que**
le volume poreux de la vitrocéramique est supérieur à 150 mm³/g et inférieur à 1500 mm³/g.

20. Vitrocéramique poreuse selon l'une quelconque ou plusieurs des revendications 16 à 19,
**caractérisée en ce que**
la vitrocéramique contient des oxydes des métaux du 1^{er} au 4^{ème} groupe principal ainsi que des oxydes des métaux des groupes secondaires.

21. Vitrocéramique selon la revendication 20,
**caractérisée en ce qu'**
on choisit l'oxyde cristallin dans le groupe constitué de TiO₂, ZrO₂, BaO, WO₃ et Fe₂O₃.

22. Vitrocéramique poreuse selon la revendication 20-21,
**caractérisée en ce que**
les oxydes sont présents, dans la vitrocéramique, individuellement ou en mélange entre 1 et 50 % en poids.

23. Procédé de fabrication de la vitrocéramique poreuse selon la revendication 16,
**caractérisé en ce qu'**
on fabrique la vitrocéramique en mélangeant un sol de SiO₂ aqueux et un ou plusieurs sols d'oxydes des métaux du 1^{er} au 4^{ème} groupe principal ainsi que d'oxydes des métaux des groupes annexes.

24. Procédé selon la revendication 23,
**caractérisé en ce qu'**
on ajoute au sol total une solution aqueuse d'un sel.

25. Procédé selon la revendication 23 et/ou 24,
**caractérisé en ce qu'**
on ajoute au mélange de sols 0 à 10 % en poids (par rapport au mélange de sols) de solvants organiques.

26. Procédé selon l'une quelconque ou plusieurs des revendications 23 à 25,
**caractérisé en ce qu'**
on sèche le mélange de sols par pulvérisation.

27. Procédé selon la revendication 26,
**caractérisé en ce qu'**
on calcine le mélange de sols séché par pulvérisation à une température supérieure à 800°C et inférieure à 1300°C.

28. Utilisation de vitrocéramiques poreuses selon la revendication 16 dans des matériaux dentaires selon la revendication 1.

29. Utilisation de vitrocéramique poreuse selon la revendication 16 pour charger en composés polymérisables, éthyléniquement insaturés.
